# EUROPEAN PATENT APPLICATION

(11) **EP 3 220 368 A1**
(43) Date of publication of application: **20.09.2017**
(21) Application number: 17159469.0
(22) Date of filing: 06.03.2017
(51) Int. Cl.: G08B 21/06, A61B 5/18, B60K 28/06

(54) **SYSTEM AND METHOD FOR PROVIDING CONTEXT-SPECIFIC VEHICULAR DRIVER INTERACTIONS**

(30) Priority: 15.03.2016 US 201615070516
(71) Applicant: Xerox Corporation, Rochester, NY 14644 (US); Palo Alto Research Center, Incorporated, Palo Alto, CA 94304 (US)
(72) Inventor: ROBERTS, Michael, Los Gatos, CA California 95033 (US); GUNNING, David Richard, Seattle, WA Washington 98199 (US); BALA, Raja, Allen, TX Texas 75013 (US); DENT, Kyle D., San Carlos, CA California 94070 (US)
(74) Representative: Gill Jennings & Every LLP

(57) **Abstract**

Interacting with the driver based on the driver's context can keep help keep the driver alert. The context can be determined determining driver characteristics including the interests and by monitoring the circumstances surrounding the driver, such as the state of the driver using sensors included in the vehicle, the state of the vehicle, and the information about the driver's current locale. The characteristics and the monitored circumstances define the context of driver. Information of interest to the driver is obtained and is used to generate actions that are recommendable to the driver based on the driver's context. The actions are used to keep the driver alert.

## Description

### Field

This application relates in general to vehicular safety, and in particular, to a system and method for providing context-specific vehicular driver interactions.

### Background

Alert drivers are an essential requirement for safe roads. Unfortunately, due to constant demands of modern life, such as insufficient time for sleep, long work hours, and long commutes, many people drive even when they are too tired to focus on the road, not having the option to stay off the roads despite their tired state. Thus, one poll in the United States has estimated that 60% of adult drivers have driven while feeling drowsy, and more than one third has actually fallen asleep at the wheel in the past year. Such drivers may fail to react in time to road conditions, other vehicles, and pedestrians on the roads, and are at an increased risk of being in a potentially-fatal car accident. Such risk further increases if a driver falls asleep at the wheel entirely. Consistently, the National Highway Traffic Administration conservatively estimates that at least 100,000 police-reported crashes in the United States are a result of driver fatigue, resulting in an estimated 1,550 deaths.

Multiple technologies exist that attempt to prevent the fatigue-related crashes, though none of them are ideal. For example, the Nad-Zapper™ Anti-Sleep Alarm includes a motion-detector wearable behind a driver's ear that sounds an alarm when the driver's head tilts forward at a certain speed, waking the driver. However, the alarm is activated only after the driver falls asleep and is at risk of losing control of the vehicle, thus failing to prevent the dangerous situation of the driver falling asleep from taking place. Further, until the next episode of falling asleep, the alarm does nothing to keep the driver awake.

Similarly, other systems, such as the driver alert system produced by Ford^{®} Motor Company of Dearborn Michigan, evaluate variations in lateral position of the vehicle, steering wheel angle, and velocity to determine if the driver has lost control of the vehicle. However, such technologies do not detect micro-sleep, sleep which lasts only a few seconds, while the car is on a straight road and may not attempt to awaken the driver before an accident occurs. Further, such systems do not attempt to keep the driver who needs to stay on a road awake before the driver actually loses control of the vehicle.

Likewise, other systems, such as a system researched by Volvo^{®} Group of Gothenburg, Sweden, perform a visual analysis of the driver using cameras in the vehicle and process images to detect signs of drowsiness in the driver's face. Upon detecting signs of drowsiness, such systems provide a warning to the driver that the driver is drowsy on the assumption that the driver will take a break sufficient enough to rest. Such systems do no directly increase the driver's alertness and as rest may not be possible for a driver in certain situations, the warnings of such systems may be ignored by the driver.

Accordingly, there is a need for a way to measure a level of a driver's alertness and increase that level of alertness to allow when the driver is drowsy.

### Summary

Interacting with the driver based on the driver's context can help keep the driver alert. The context can be determined by determining driver characteristics including driver interests and by monitoring the circumstances surrounding the driver, such as the state of the driver using sensors included in the vehicle, the state of the vehicle, and the information about the driver's current locale. The characteristics and the monitored circumstances define the context of driver. Information of interest to the driver is obtained and is used to generate actions that are recommendable to the driver based on the driver's context. The actions are used to keep the driver alert.

In one embodiment, a system and method for performing context-specific actions towards a vehicular driver are disclosed. A context of a driver of a vehicle is determined, including: determining a state of the driver; determining a state of the vehicle; and determining one or more characteristics of the driver. One or more actions are recommended to be performed by the system with respect to the driver based on the context. One or more of the recommended actions are performed.

Still other embodiments of the present invention will become readily apparent to those skilled in the art from the following detailed description, wherein is described embodiments of the invention by way of illustrating the best mode contemplated for carrying out the invention. As will be realized, the invention is capable of other and different embodiments and its several details are capable of modifications in various obvious respects, all without departing from the spirit and the scope of the present invention. Accordingly, the drawings and detailed description are to be regarded as illustrative in nature and not as restrictive.

### Brief Description of the Drawings

FIGURE 1 is a block diagram showing a system for performing context-specific actions towards a vehicular driver in accordance with one embodiment.
FIGURE 2 is a flow diagram showing a method for performing context-specific actions towards a vehicular driver in accordance with one embodiment.
FIGURE 3 is a flow diagram showing a routine for determining the driver's context for use in the method of FIGURE 2 in accordance with one embodiment.
FIGURE 4 is a flow diagram showing a routine for monitoring the driver state for use in the routine of FIGURE 3 in accordance with one embodiment.
FIGURE 5 is a flow diagram showing a routine for performing fine gaze estimation for use in the routine of FIGURE 4 in accordance with one embodiment.
FIGURE 6 is a flow diagram showing a routine for determining driver characteristics for use in the method of FIGURE 2 in accordance with one embodiment.
FIGURE 7 is a flow diagram showing a routine for recommending an action to be taken with respect to the driver for use in the method of FIGURE 2 in accordance with one embodiment.

### Detailed Description

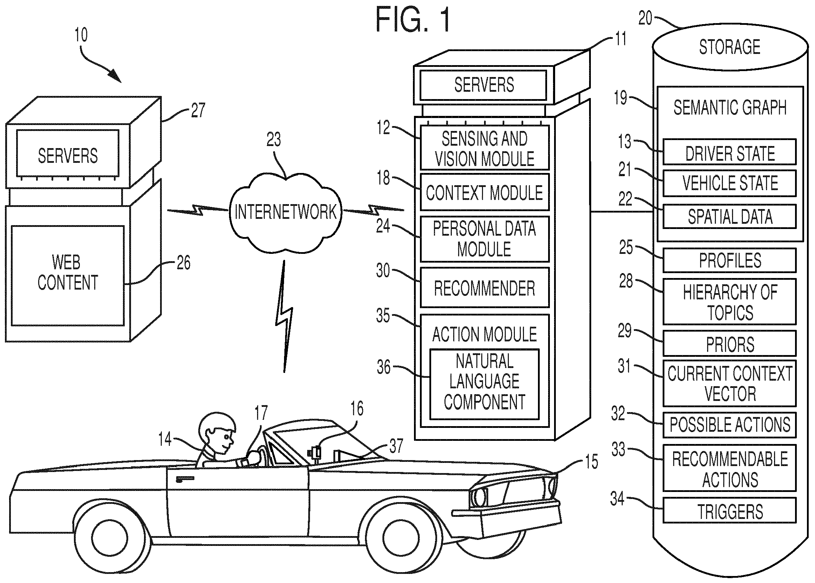

Driver alertness, and consequently, safety on the roads, can be improved by customizing interactions with the driver to be based on the driver's current context. FIGURE 1 is a block diagram showing a system 10 for performing context-specific actions towards a vehicular driver in accordance with one embodiment. The system 10 includes one or more servers 11 that execute a sensing and vision module 12 responsible for monitoring a state 13 of a driver 14 of a vehicle 15. The state of the driver can describe whether the driver is alert or drowsy, though other kinds of states are possible. For example, in a further embodiment, driver state 13 can include the driver's emotions. The sensing and vision module 12 interacts with one or more sensors inside the vehicle 15 to monitor the state. The sensors include at least one driver-facing digital camera 16 that monitors the driver. The camera 16 can be a camera 16 that records visible light images; alternatively, the camera 16 can also be an infrared camera 16. Other kinds of cameras are also possible. In addition, other sensors can be monitoring the user. For example, biometric sensors can be worn by the driver, such as being integrated into a smartwatch 17, and can sense physiological data of the driver 14. Thus, the sensor can be a pulse oximeter integrated into the smartwatch 17 that can record a photoplethysmogram ("PPG wave") of the driver. Still other kinds of sensors are possible.

The servers 11 further execute a context module 18 that determines that driver's context, a set of circumstances associated with the driver at a particular moment of time. The context is the represented in a context graph 19 that is stored in the context module 18 and that can be additionally stored in backup storage media 20. The context graph 19 is a semantic graph 19 (the context graph 19 is here forth referred to in the description below as a "semantic graph 19"). In addition to the state of the driver 14, the semantic graph 19 reflects the state 21 of the vehicle 15, such as the speed of the vehicle or repetitions per minute of the vehicle's engine, any objects in front of the car, conditions of the road on which the vehicle 15 is driving, and whether vehicle maintains the position with regards to lanes on a road (which can be determined using sensors within the vehicle), though other kinds of vehicles state information is also possible. In addition, the storage 20 can include spatial data 22 regarding the driver's locale, such as a particular city or county, though other kinds of geographical locales are possible. The spatial data 22 can include data about restaurants, shops, and other points of interest in the locale. The spatial data 22 can be obtained by the context module 18 from one or more webpages 21 accessible through an Internetwork 23, such as the Internet or a cellular network. The context module 18 can receive the vehicle's current location, which can be obtained using a GPS receiver built into the vehicle and transmitted via the wireless transceiver built into the vehicle 15 that can connect to the Internetwork 23, and taken identify those points of interest that are proximate to the drivers of location. The context module 18 can include the points of interest in to the semantic graph 19.

In a further embodiment, the sensors of the vehicle 15, such as the camera or other sensors, can measure a driver's load (not shown), such whether the load is high and the driver needs to focus attention on driving and should not be distraction; a normal load, when some driver attention is required for driving and addition load for conversation is permitted; or low, when the driver is parked or idling at a stoplight. The load can be measured by evaluating the eye blinking rate (which can be determined as described below), by measuring saccade, or pupil dilation, though other ways to evaluate the driver load are possible. The data from the sensors can be transmitted by the wireless transceiver to the sensors and incorporated into the semantic graph 19. The semantic graph 19 can be generated from the driver state 13, the vehicle state 21 , the locale information 22, and other information, as described in commonly-assigned U.S. Patent No. 9,208,439, to Roberts et al., issued December 8, 2015, the disclosure of which is incorporated by reference. Other ways to create the semantic graph 19 are possible.

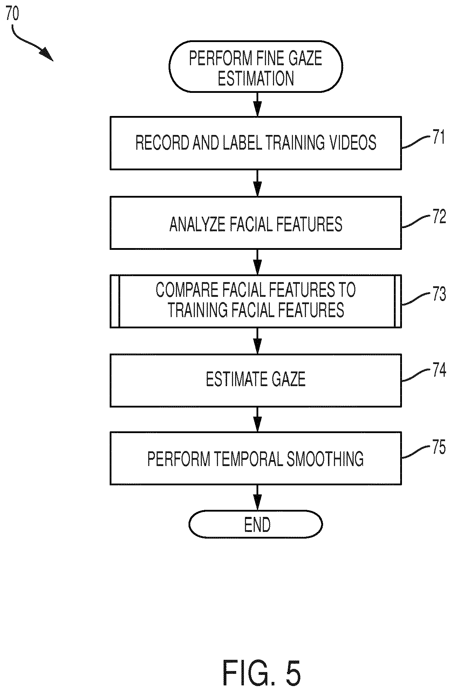

The servers 11 further execute a personal data module 24 that collects information about the driver and, based on the collected information, learns characteristics of the driver, such as current and historical interests of the driver, though other characteristics of the driver are possible; the characteristics are represented profile 25 of the driver, as further described below with reference to FIGURE 5. Briefly, the personal data module extracts 23 data items from web content 26 associated with the driver that can be retrieved by the servers 11 via the Internetwork 23. The web content 26 can include information feeds, such as social networking posts by the driver or by the driver's social network connections, RSS feeds that the driver is subscribed to, the driver's social network profiles maintained on one or more servers 27, though other kinds of web content 26 are possible.

The extracted data items are compared by the personal data module 24 to a hierarchy of topics 28 (which can include topics grouped in different categories) and can be stored the storage 20, though other kinds of comparisons are possible. Based on the comparison, the topics in the hierarchy 28 that are associated with each data item are identified. Based on the identified topics, the data items can be classified in the uniform parameter space. In particular, in one embodiment, a representational vector can be generated from the identified topics for each of the data items. Such a vector describes the classification of the document in terms of the hierarchical topics and defines a point in high dimensional vector space unique to the content of that data item. The vectors can be weighed based on the age of the data item, with vectors for more recent data items been weighed more heavily. The weighed vectors are combined into a single vector that functions as a profile 25 of the driver in this description vector that describes the driver's current and historical interests. The fields in the vector correspond to numeric values related to the topics included in the hierarchy 28. In a further embodiment, the personal data module 24 can combine the vectors associated with multiple users to form population priors 29.

The priors 29 can be created through techniques such as clustering and unsupervised learning, though other techniques are also possible. For example, the priors 29 can be constructed through collaborative filtering rule, which can be used to make recommendations to combine the profiles 25 based on similarity. Similarly the population priors could be constructed based on the other information associated with the drivers 13, such as the age of the driver, and other data in their profile 25. Still other ways to create the population priors are possible. The population prior closest to a profile 25 of a particular driver can be used instead of the driver profile 25 for recommending actions, as further described below.

The semantic graph 19 and the profile 25 (or a closest prior) of a driver 14 are merged together by a recommender 30 executed by the servers 11 into a single vector space "current context" vector 31 representing the driver's current context, which covers both the driver's personal characteristics (such as his interests), the driver's state, vehicle state, and the locale. Further, the recommender 30 has access to a list of possible actions 32 that could be taken with respect to the driver 14. Such actions can include particular conversation patterns to be executed with the driver 14 and other actions. For example, such actions can include: conversing with the driver 14 on topics such as a social networking post made by a social networking connection of the driver 14; asking the driver 14 if the driver 14 would like to have a news story read to him or her, to hear about a particular point of interest nearby. Still other possible actions in the list 32 are possible. The possible actions are used to generate parameterized, recommendable actions 33 that can be recommended for implementation.

To generate the recommendable actions 33, the recommender 30 extracts recent data items representing current information associated with the driver from the web content 26, such as the recent social network of connections of the driver 14, recent news stories, and uses the extracted content to parameterize the possible actions. The actions 33 are further generated based on current context vector 31. Thus, for example, if a possible action 32 is having a conversation with the driver, the driver's interest indicated in the vector 31 include cooking, and the extracted current information includes a social networking post about cooking, a generated action could be a conversation about the extracted social networking post.

Each of the generated recommendable actions are represented by a characterization vector that describes the action in high-dimensional vector space. The vector space corresponds to a representation of the hierarchy of topics 28. For example, talking about a point of interest that is a Chinese/Asian Fusion restaurant might have high values in its description vector for "Asian Fusion Cuisine" and "Chinese Cuisine." Similarly, a piece of content intended-to-be-played when the user is drowsy might have a high value for "drowsy." If such a piece of content were also relevant to posts made by a close friend, the piece of content might also have a "CloseFriend" value close to 1, as opposed to 0 for a non-close friend item.

Further, at least some of the generated actions 32 can be associated with a triggering condition 34 that must be fulfilled before the action is implemented, as further described below. For example, an action that includes conversing with the driver 14 may not be implemented until being triggered by the driver's cognitive load being low enough to safely conduct the conversation. Similarly, conversing about a particular point of interest can be triggered by the point of interest being nearby.

The recommender 30 analyzes characterization vectors of the generated actions and ranks them. The ranking can be performed in a plurality of ways. For example, the characterization vectors can be compared based on their closeness to the driver profile 25. For example, in one embodiment, the values in the slots of the characterization vectors are multiplied by the values in the corresponding slots in the vector that is the driver's profile 25, and then these values are summed to give a score for the item. The recommender 30 compares the scores for the different vectors and ranks the vectors for the recommendable actions 33 based on the comparison. In a further embodiment, the actions can be ranked based on novelty, which describes whether the action has been done before, and recency, which describes how recently a particular action has been done before. In a still further embodiment, multiple rankings using multiple techniques can performed, with the results being differentially weighed and combined. The weights can be optimized using machine learning.

Actions 33 whose vectors are of a certain rank, such as the top two scoring vectors, are recommended by the recommender 30 for execution. In a further embodiment, other actions 33 of other ranks could be also used. The generation and recommendation of the data items can be done as described in commonly-assigned U.S. Patent Application No. 2015/0142785, published May 21, 2015, by Roberts et al., the disclosure of which is incorporated by reference, and as described in "Activity-based serendipitous recommendations with the Magitti mobile leisure guide", by Belotti et al., CHI 2008, 5 April 2008, the disclosure of which is incorporated by reference.

The recommended actions 33 are implemented by an action module 35 implemented by the servers 11. The action module 35 includes a natural language component 35 that engages into natural language conversations with the driver 14. The conversation can be performed as described in commonly-assigned U.S. Patent Application Publication No. 2015/0293904, published October 15, 2015. The recommender 30 can provide the recommended actions 33 to the action module 35 in a variety of forms, such as a form of a serialized JSON objects, which, in addition to the description of the actions 33, the current state 13 of the driver 14, and any information necessary to implement the action. Thus, the provided information can include triggers 34 for taking the action at relevant engagement points (such a driver being drowsy and nearby point of interest); contextual information about driver's state of alertness (with the information being updated as the information changes in real-time) retrieved from the semantic graph 19; personal data from the profile 25 of the driver 14, and extracted web content 26 such as social networking updates and sports and entertainment which can be used to implement the recommended actions 33. The action module analyzes the driver state 13 and other provided information to recognize when a triggering condition 34 has taken place and performs a recommended action associated with the triggering condition 34 that has occurred.

The natural language component 35 can support both conversation prompts and dialog acts. A conversation prompt causes the component 36 to invoke one of the predefined patterns like reading a social networking post or playing a game. Dialog acts provide for simpler interactions from other modules such as confirming a musical selection. For example, if a recommendation is made for an upbeat tune to keep a driver from feeling drowsy, the recommender 30 can issue a request to *ConfirmTune(X)* where *X* is the recommended tune given the driver's current state and known preferences. The request causes the natural language component 36 to ask the question of the driver and provide the driver's acknowledgement or denial of the suggested music.

The action module 36 interacts with the driver 14 through a driver interface 37 located in the vehicle through the Internetwork 23 to perform the recommended action. In one embodiment, the driver interface 37 can be a software component and utilize onboard computer systems that are integrated into the vehicle 15, such as a trip or a navigational computer, rearview monitors, and other components. In a further embodiment, the driver interface 37 can include hardware components that are exclusively part of the system 10 and not used by other onboard vehicle components. The driver interface 37 can include a visual display, such as in a form of an animated ring that changes shape and opacity when the interface delivers speech, though other visual representations of the interface are also possible. Upon decision of the action module 35 to engage in a particular conversation, the action module 35 transmits the text to be spoken to the driver 14 to the agent interface 37 within the vehicle, which performs text-to-speech ("TTS") conversion, such as using a commercially using the available TTS software like Nuance^{®} produced by Nuance Communications, Inc. of Burlington, Massachusetts, though other ways to perform the text-to-speech conversion. The received speech is a natural language speech. The speech is delivered through speakers (not shown) integrated into the vehicle 15. Driver 14 responses are picked up through one or more microphones connected to the driver interface 13, and can be used to further interact with the driver 14. The interface 37 performs basic thresholding and other needed audio processing on the driver speech before performing speech-to-text conversion using an appropriate speech-to-text conversion software, and sending the text to the natural language component 36 for analysis and for possibly continuing the conversation or taking another action. The interface 37 can also include other components for taking actions, such as a light that can be flashed at the user to wake the user up. Other components in the driver interface 37 are further possible.

As mentioned above, the servers 11 include multiple modules for carrying out the embodiments disclosed herein. The modules can be implemented as a computer program or procedure written as source code in a conventional programming language and is presented for execution by the central processing unit as object or byte code. Alternatively, the modules could also be implemented in hardware, either as integrated circuitry or burned into read-only memory components, and each of the servers can act as a specialized computer. For instance, when the modules are implemented as hardware, that particular hardware is specialized to perform the communications described above and other computers cannot be used. Additionally, when the modules are burned into read-only memory components, the computer storing the read-only memory becomes specialized to perform the operations described above that other computers cannot. The various implementations of the source code and object and byte codes can be held on a computer-readable storage medium, such as a floppy disk, hard drive, digital video disk (DVD), random access memory (RAM), read-only memory (ROM) and similar storage mediums. Other types of modules and module functions are possible, as well as other physical hardware components. For example, the servers 11 can include other components found in programmable computing devices, such as input/output ports, network interfaces, and non-volatile storage, although other components are possible. The servers 11 and the storage 20 can be a part of a cloud-computing environment or be dedicated servers.

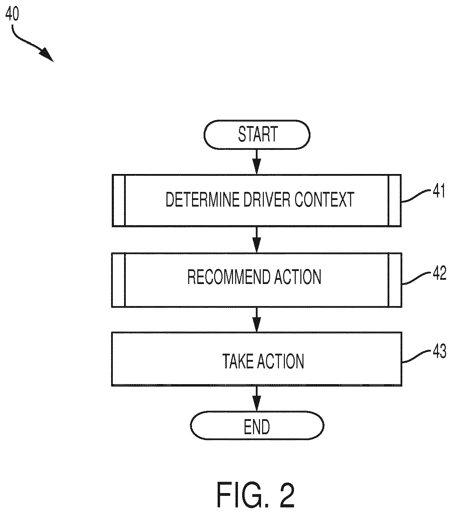

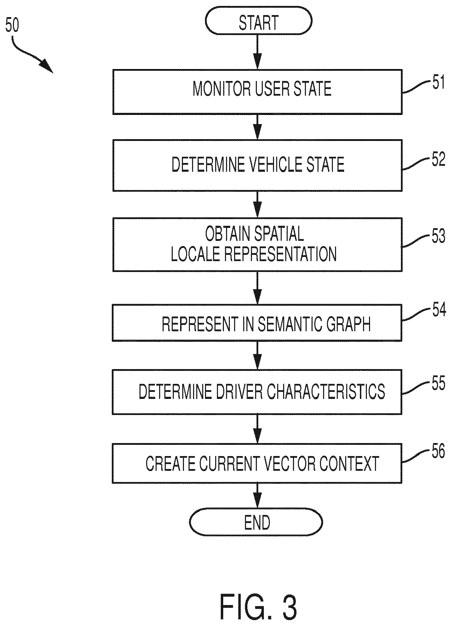

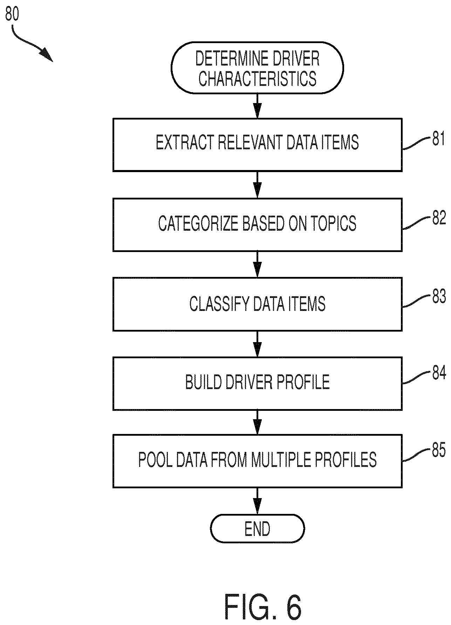

Tailoring actions to be taken towards a driver based on his context and interests helps to keep the driver alert and driving safely. FIGURE 2 is a flow diagram showing a method 40 for performing context-specific actions towards a vehicular driver in accordance with one embodiment. The method 40 can be implemented using the system of FIGURE 1. Current driver context is determined, as further described below with reference to FIGURE 3 (step 41). One or more actions to be taken with respect to the driver is recommended, as further described below with reference to FIGURE 6. One or more of the recommended actions are performed, terminating the method 40 (step 43). As described above with reference to FIGURE 1, the recommended actions can include engaging the driver in conversation, or performing other actions to keep the driver engaged. As described above with reference to FIGURE 1, at least some of the recommended items can be associated with a trigger, and are executed when a trigger associated with the recommended action is recognized. Some actions may also not be associated with a trigger and be executed upon being recommended.

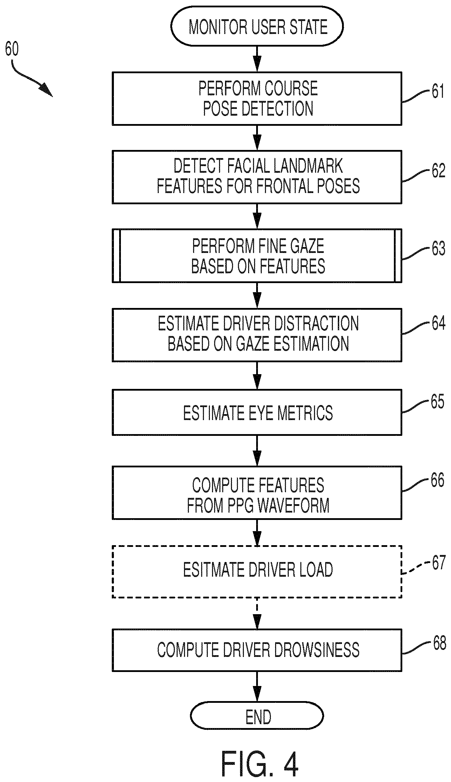

A context of a driver can include multiple components. FIGURE 3 is a flow diagram showing a routine 50 for determining the driver's context for use in the method of FIGURE 2 in accordance with one embodiment. The state of the driver is determined using one or more sensors included in the vehicle, such as a camera, though other ways to determine the context are possible, such as further described below with reference to FIGURE 4 (step 51). The state of the vehicle is determined using sensors in the vehicle (step 52). Spatial information about the locale in which the driver is currently located are obtained, such as by retrieving the information from the Internet (step 53). The state of the driver, the state of the vehicle, and the spatial information are represented in a semantic graph (step 54). Driver characteristics, including the profile of the driver, are determined, as further described below with reference to FIGURE 6 (step 55); in a further embodiment, priors of profiles could be created (not shown). A vector characterizing the current context, covering both driver characteristics, driver state, vehicle state, and current locale information, is created by merging the semantic graph with the driver profile (or a prior closest to the driver profile) (step 56), as further described below with reference to FIGURE 5, terminating the routine 50.

FIGURE 4 is a flow diagram showing a routine 60 for monitoring the driver state for use in the routine of FIGURE 3 in accordance with one embodiment. Initially, a course pose estimation (left-front-right) is performed by simultaneously running frontal, left, and right face detectors on an image captured by the camera in the vehicle (step 61). For those frames that are detected as frontal face pose, a set of facial landmark features is detected and tracked over time by a technique such as an application of a Kalman filter, though other techniques are possible (step 62). The features can include relative locations of face parts, such as eyes, nose, and mouth, though other features are also possible. Fine gaze estimation of the driver looking in common directions is performed based on the features with the driver's features being used to estimate where the driver is looking at, as further described with reference to FIGURE 5 (step 63). In one embodiment, the gaze estimation is performed with regards to eight different directions, though other numbers of directions are possible in a further embodiment. The results of the fine gaze estimation are combined with other contextual information, such as driver route and vehicle speed to obtain a measure of a level of driver distraction (step 64). Eye metrics, such as blink rate and percentage of eye closure are estimated using the camera (step 65). Head motions, such as frequency of nodding and drooping motions, are estimated, such as using techniques described in E. Murphy-Chutorian, M. Trivedi, "Head Pose Estimation in Computer Vision: A Survey", IEEE TPAMI, vol. 31, no. 4, pp. 607-626, 2009, and E. Murphy-Chutorian, A. Doshi, M. M. Trivedi, "Head Pose Estimation for Driver Assistance Systems: A Robust Algorithm and Experimental Evaluation", IEEE ITSC, 2007, the disclosures of which is incorporated by reference, though other techniques are also possible (step 66). The output of the PPG waveform sensor worn by the driver is obtained and features of the PPG waveform, such as peak-to-peak statistics and power spectral density, are computed, such as further described in B. Lee and W. Chung, "Driver alertness monitoring using fusion of facial features and bio-signals", IEEE Sensors Journal, Vol. 12, No. 7, pp. 2416-2422, the disclosure of which is incorporated by reference, though other ways to compute the features are possible (step 67). The computed PPG waveform, estimated eye metrics, and estimated head motions are combined with other contextual information, such as the time of day and other relevant information about the driver, such as the driver having just returned from a long journey from a different time zone (which can be processed and included into the semantic graph and which can be determined based on sensors in the driver's vehicle or by analyzing social networking posts, though other ways to determine such information are possible)to provide a measure of driver drowsiness (step 68). Optionally, the estimated metrics can be displayed to the driver or be provided to a third party through the connection to the Internetwork (step 69), terminating the routine 60. In a further embodiment, other data can be analyzed using the sensors in the vehicle, such as the driver's gestures or the environment surrounding the driver.

Estimating the directions in which the driver looks can help to determine how drowsy the driver is. FIGURE 5 is a flow diagram showing a routine 70 for performing fine gaze estimation for use in the routine 60 of FIGURE 4 in accordance with one embodiment. One or more videos of the driver looking into known directions are recorded and labeled with the known direction (step 71). The videos can be stored in the storage. Facial features of the driver in each of the training videos are identified (step 72). The facial features of the driver obtained in step 62 are compared to the facial features in the training videos (step 73) and the fine gaze estimation is performed based on the comparison. Temporal smoothing is performed on the gaze estimates to obtain coherency and consistency across neighboring frames (step 74), terminating the routine 70.

FIGURE 6 is a flow diagram showing a routine 80 for determining driver characteristics for use in the method 40 of FIGURE 2 in accordance with one embodiment. A user model that includes data items associated with the driver, driver interests, social networking connections, are extracted from web content, such as social networks to which the driver belongs or other websites to which the driver belongs that host user generated content (step 81). The extracted data items are compared to a hierarchy of topics, and the topics in the hierarchy that are associated with each data item are identified (step 82). The data items can be classified in the uniform parameter space using the identified topics (step 83). As described above with reference to FIGURE 1, in one embodiment, the classification can include generating a representational vector of the identified topics. The vectors for each of the data items are weighed based on the age of the data item, giving more weight to the vectors of the more current data items (representing the driver's current characteristics) than older data items (step 84). The weighed vectors are combined into a single vector that functions as a profile of the driver that describes the driver's current and historical characteristics, such as interests (step 85). Optionally, the personal data and the profile vectors can be combined to form population priors (step 86), terminating the routine 80.

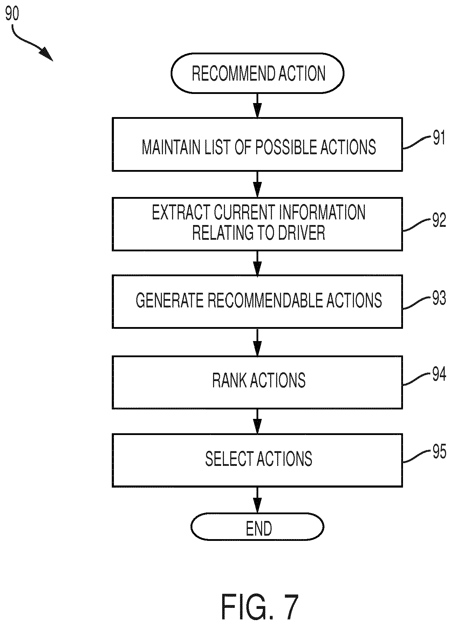

Based on the driver's context and the driver's characteristics, an action can be recommended for execution with regards to the driver. FIGURE 7 is a flow diagram showing a routine 90 for recommending an action to be taken with respect to the driver for use in the method 40 of FIGURE 2 in accordance with one embodiment. A list of possible actions is maintained (step 91). Current information relating to the driver, such as recent social networking posts of the driver's social networking connections, are extracted from the web, and optionally, indexed in the semantic graph 19 (step 92). Recommendable actions are generated based on the current extracted information, the potential items, and the current context vector (step 93). The recommendable actions are ranked (step 94). As mentioned above with reference to FIGURE 1, multiple ways to perform the ranking are possible. One or more actions are selected for execution based on the rank, as described above with reference to FIGURE 1 (step 95), terminating the routine 90.

While the invention has been particularly shown and described as referenced to the embodiments thereof, those skilled in the art will understand that the foregoing and other changes in form and detail may be made therein without departing from the spirit and scope of the invention.

## Claims

1. A system for performing context-specific actions towards a vehicular driver, comprising:
one or more servers connected over an Internetwork to a vehicle, the servers configured to execute code, comprising:
a context module configured to determine a context of a driver of the vehicle, comprising:
a driver state module configured to determine a state of the driver; and
a vehicle state module configured to determine a state of the vehicle;
a characteristic module configured to determine one or more characteristics of the driver;
a recommendation module configured to recommend one or more actions to be performed to the driver based on the context; and
a performance module configured to perform one or more of the recommended actions.

2. A system according to Claim 1, further comprising:
a location module configured to determine a location of the vehicle;
a data module configured to obtain data regarding the determined location.

3. A system according to Claim 2, further comprising:
a graph module configured to represent the driver state, the vehicle state, and the location data in a semantic graph;
a vector module configured to represent the characteristics in a vector; and
a merging module configured to merge the semantic graph and the vector into a different vector representing the context.

4. A system according to Claim 1, further comprising:
a pose module configured to perform a course pose estimation on the driver using a camera included in the vehicle;
a feature module configured to detect one or more facial landmark features on the driver's face using at least some of results of course pose estimation;
a gaze module configured to perform a fine gaze estimation on the driver using the detected facial landmark features comprising determining one or more directions of the driver's gaze; and
a distraction module configured to use the fine gaze estimation and state of the vehicle to measure a level of distraction of the driver.

5. A system according to Claim 4, further comprising:
eye metric module configured to estimate one or more eye metrics of the driver, the metrics comprising at least one of a blink rate and percentage of eye closure, using the camera;
a biometric data module configured to obtain biometric data using a sensor wearable by the driver; and
a drowsiness module configured to use the biometric data, the eye metrics, and the head motion to measure a level of the driver's drowsiness.

6. A method for performing context-specific actions towards a vehicular driver, comprising the steps of:
determining a context of a driver of a vehicle, comprising:
determining a state of the driver; and
determining a state of the vehicle;
determining one or more characteristics of the driver;
recommending one or more actions to be performed to the driver based on the context; and
performing one or more of the recommended actions,
wherein the steps are performed by at least one suitably programmed computer.

7. A method according to Claim 6, wherein determining the context further comprises:
determining a location of the vehicle;
obtaining data regarding the determined location.

8. A method according to Claim 7, further comprising:
representing the driver state, the vehicle state, and the location data in a semantic graph;
representing the characteristics in a vector; and
merging the semantic graph and the vector into a different vector representing the context.

9. A method according to Claim 6, further comprising:
performing a course pose estimation on the driver using a camera included in the vehicle;
detecting one or more facial landmark features on the driver's face using at least some of results of course pose estimation;
performing fine gaze estimation on the driver using the detected facial landmark features comprising determining one or more directions of the driver's gaze; and
using the fine gaze estimation and state of the vehicle to measure a level of distraction of the driver.

10. A method according to Claim 9, further comprising:
estimating one or more eye metrics of the driver, the metrics comprising at least one of a blink rate and percentage of eye closure, using the camera;
obtaining biometric data using a sensor wearable by the driver; and
using the biometric data, the eye metrics, and the head motion to measure a level of the driver's drowsiness.
